# EUROPEAN PATENT APPLICATION

(11) **EP 4 245 771 A1**
(43) Date of publication of application: **20.09.2023**
(21) Application number: 21877964.3
(22) Date of filing: 05.10.2021
(51) Int. Cl.: C07K 14/78, C07K 14/47, A61K 47/62, A61P 35/00, A61K 49/00, A61K 51/08, A61K 38/00

(54) **NOVEL PROTEIN SPECIFICALLY BINDING TO CALRETICULIN AND HAVING HUMAN FIBRONECTIN DOMAIN III SCAFFOLD AND USE THEREOF**

(30) Priority: 06.10.2020 KR 20200128843
(71) Applicant: Industry Foundation of Chonnam National University, Gwangju 61186 (KR)
(72) Inventor: HONG, Yeongjin, Gwangju 61682 (KR); MIN, Jung-Joon, Gwangju 61461 (KR); KIM, Dong-Yeon, Hwasun-gun Jeollanam-do 58126 (KR); PYO, Ayoung, Gwangju 62003 (KR); YOU, Sung-Hwan, Gwangju 61419 (KR); THANGAM, Ramar, Hwasun-gun Jeollanam-do 58115 (KR)
(74) Representative: Dehns
(86) International application number: PCT/KR2021/013640
(87) International publication number: WO 2022/075713

(57) **Abstract**

The present invention provides a novel protein specifically binding to calreticulin and having the scaffold of human fibronectin domain III and a use thereof, wherein the protein can be used for diagnosing cell damage in an early stage to early determine cancer therapy effects and rapidly evaluate *in vivo* toxicity, thus finding advantageous applications as a candidate material for probes for in vivo diagnosis and treatment of cancer.

## Description

### TECHNICAL FIELD

The present invention relates to a novel protein specifically binding to calreticulin and a use thereof, and more particularly, to a novel protein specifically binding to calreticulin and having the scaffold of human fibronectin domain III and a use thereof.

### BACKGROUND ART

The nuclear medicine image, which is a molecular imaging technique, is capable of imaging in vivo the distribution of various biochemical substances and quantitative measuring physiological indicators in the body, and thus is advantageously used for investigating biochemical or pathological phenomena, diagnosing diseases, determining prognosis after treatment, planning for treatment, and the like. In addition, the anatomical information provided by nuclear medicine imaging devices lacks and the resolution is low due to the biochemical and physical principles, and thus fusion imaging devices fused with X-ray, CT or MRI, which show the structure of our body with high resolution, play an important role, and in this light, the market size of nuclear medicine imaging devices will continue to grow in the future. In particular, the increase in demand for PET/CT is leading the market growth, which means that the development of radioactive compounds that can be used in new diagnostic/treatment technologies allows for the growth into a world-class high value-added industry. However, the current radiopharmaceutical that can be used to determine the therapeutic effect on cancer in clinics is only 2-deoxy-2-[¹⁸F]fluoro-D-glucose ([18F]FDG), but it takes several months to confirm the therapeutic effect, and its use is very limited due to uptake of non-tumor areas (muscle, inflammation, central nervous system). Therefore, the need to develop a radioactive compound for the purpose of evaluating therapeutic effects and toxicity within a few days is on the rise. In this regard, Korean Patent Laid-open Publication No. 2020-0001971 discloses a radioactive compound for treating melanoma and a use thereof.

### DISCLOSURE OF THE INVENTION

### TECHNICAL PROBLEM

However, the prior art relates to a radioactive compound for treatment with an improved ability to target melanoma, and there is no research on a substance capable of early determining cancer therapy effects and evaluating in vivo toxicity simultaneously.

The present invention has been made in an effort to solve various problems including the above problems, and an object of the present invention is to provide a novel protein specifically binding to calreticulin (CRT) and having the scaffold of human fibronectin domain III and a use thereof, wherein the protein can be used for diagnosing cell damage in an early stage to early determine cancer therapy effects and rapidly evaluate in vivo toxicity. However, the object is for illustrative purpose only and the scope of the present disclosure is not limited thereby.

### TECHNICAL SOLUTION

According to one aspect of the present invention, there is provided a recombinant monobody which specifically binds calreticulin and in which a calreticulin-binding peptide is inserted into at least one among the BC loop and the FG loop of human fibronectin domain III (Fn3).

According to another aspect of the present invention, there is provided a peptide conjugate in which an anticancer compound or an anticancer protein is bound to the recombinant monobody by a covalent or non-covalent bond.

According to further another aspect of the present invention, there is provided a pharmaceutical composition for treating cancer containing the recombinant monobody or the peptide conjugate as an active ingredient.

According to still another aspect of the present invention, there is provided a peptide conjugate in which a compound for contrast is bound to the recombinant monobody by a covalent or non-covalent bond.

According to yet another aspect of the present invention, there is provided a composition for diagnosing or contrasting cancer containing the peptide conjugate as an active ingredient.

According to still yet another aspect of the present invention, there is provided a method for diagnosing a tumor, the method including: administering the composition for diagnosing or contrasting cancer to an individual; and obtaining an image by means of the composition.

According to even another aspect of the present invention, there is provided a method for contrasting cancer tissues during the surgery of a mammal except a human, the method including: administering the composition for diagnosing or contrasting cancer to an individual; obtaining an image by means of the composition; and resecting an area in which a signal is shown by the composition.

According to still another embodiment of the present invention, there is provided a method for treating cancer, the method including administering a therapeutically effective amount of the pharmaceutical composition for treating cancer to an individual.

According to yet another aspect of the present invention, there is provided a use of a recombinant monobody which specifically binds calreticulin and in which a calreticulin-binding peptide is inserted into at least one among the BC loop and the FG loop of human fibronectin domain III (Fn3), or a peptide conjugate in which an anticancer compound or an anticancer protein is bound to the recombinant monobody by a covalent or non-covalent bond, for preparing an anticancer composition.

According to yet another aspect of the present invention, there is provided a use of a recombinant monobody which specifically binds calreticulin and in which a calreticulin-binding peptide is inserted into at least one among the BC loop and the FG loop of human fibronectin domain III (Fn3), or a peptide conjugate in which an anticancer compound or an anticancer protein is bound to the recombinant monobody by a covalent or non-covalent bond, for preparing a composition for diagnosing or contrasting cancer.

### ADVANTAGEOUS EFFECTS

The novel protein specifically binding to calreticulin having the scaffold of human fibronectin domain III of the present invention as described above is highly effective in early evaluation of cancer therapy response and evaluation of toxic effects, thus finding advantageous applications as a candidate material for probes for in vivo diagnosis and treatment of a tumor. However, the scope of the present invention is not limited by such an effect.

### BRIEF DESCRIPTION OF THE DRAWINGS

FIG. 1 is a schematic view schematically illustrating the structure of a human fibronectin domain III scaffold protein of the present invention.
FIG. 2 is a schematic view schematically illustrating a construct of a recombinant monobody which specifically binds calreticulin of the present invention.
FIG. 3 is a schematic view schematically illustrating a construct of CRT-monobody-Rluc8 of the present invention.
FIG. 4 is a gel photograph for confirming the expression the total protein and the soluble protein of recombinant monobodies specifically binding to the calreticulin prepared according to an embodiment of the present invention by loading the total protein and the soluble protein thereof on SDS-PAGE.
FIG. 5 is a gel photograph for confirming the degree of protein purification by loading the CRT-monobody of the present invention on SDS-PAGE.
FIG. 6 is a fluorescent photograph of an untreated group, which is not treated with an anticancer drug, obtained by observing the calreticulin binding force of the recombinant CRT-monobody of the present invention through a microscope.
FIG. 7 is a fluorescent photograph of an experimental group treated with mitoxantrone, an anticancer drug, which is obtained by observing the calreticulin binding force of the recombinant CRT-monobody of the present invention through a microscope.
FIG. 8 is a fluorescent photograph of an experimental group treated with gemcitabine, an anticancer drug, which is obtained by observing the calreticulin binding force of the recombinant CRT-monobody of the present invention through a microscope.
FIG. 9 is a gel photograph confirming the expression of the recombinant CRT-monobody-Rluc8 protein of the present invention.
FIG. 10 is a fluorescent photograph obtained by observing the activity of the CRT-monobody-Rluc8 fusion protein.

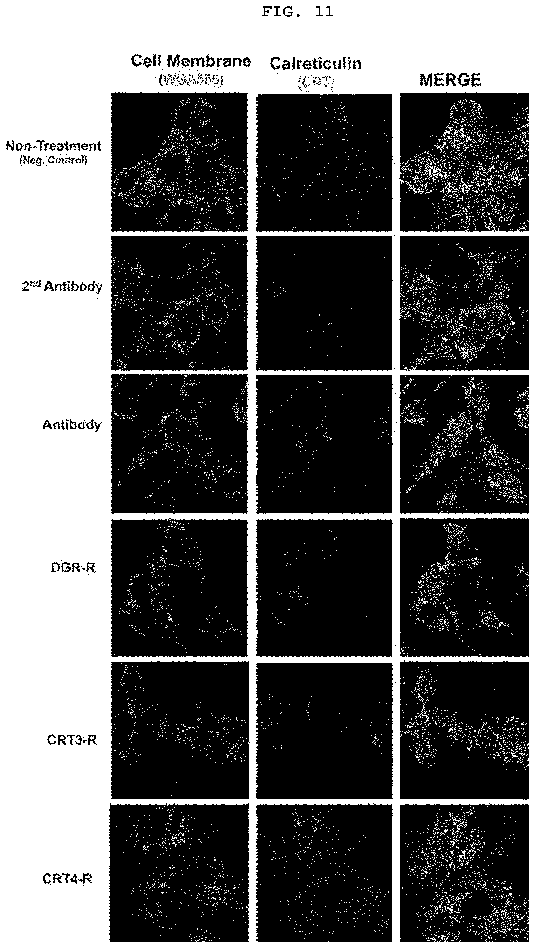

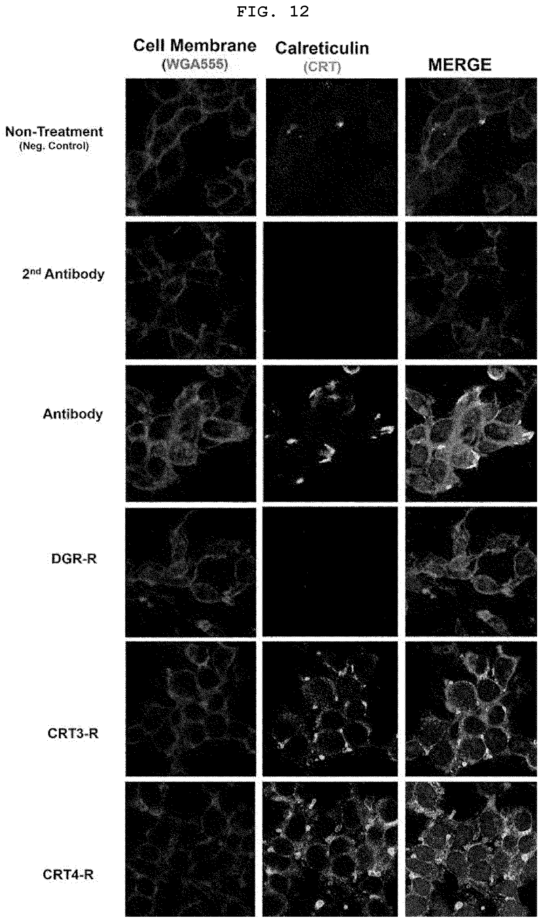

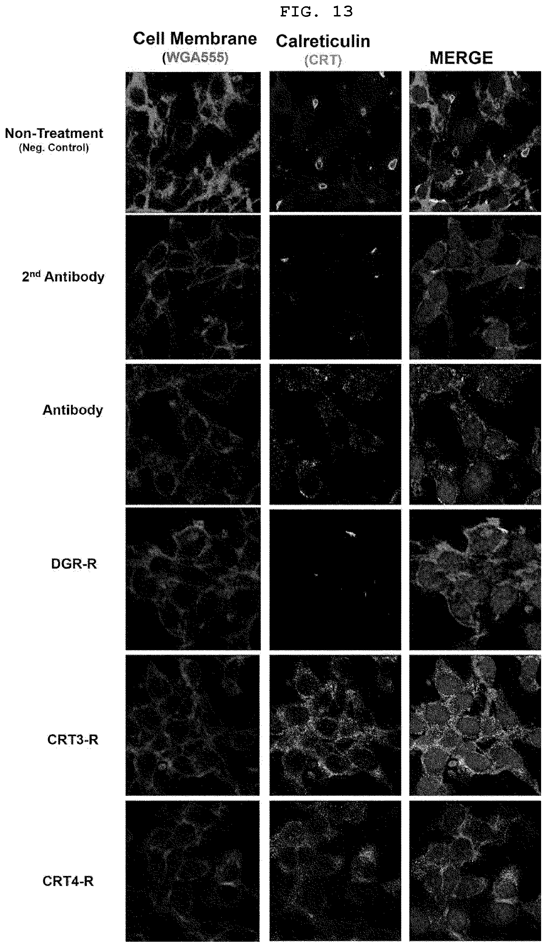

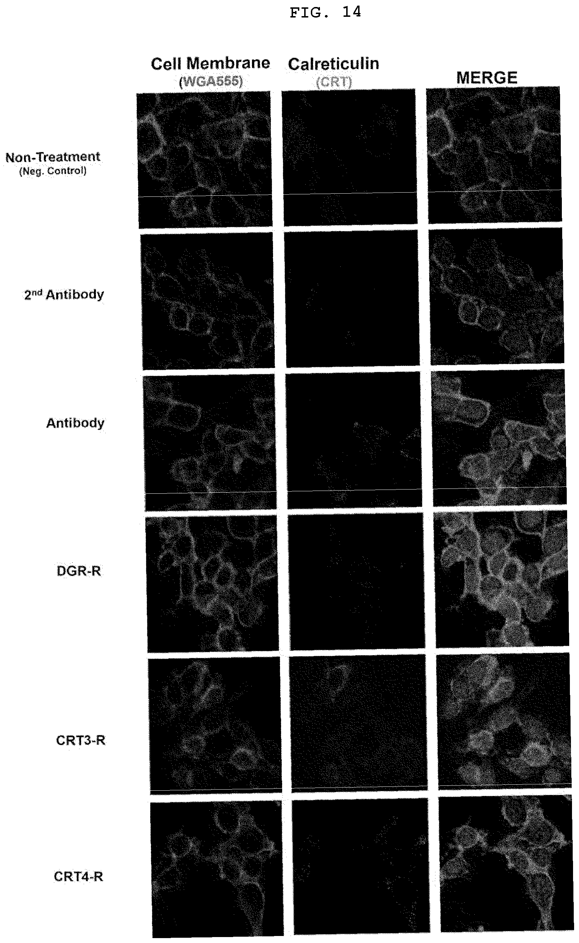

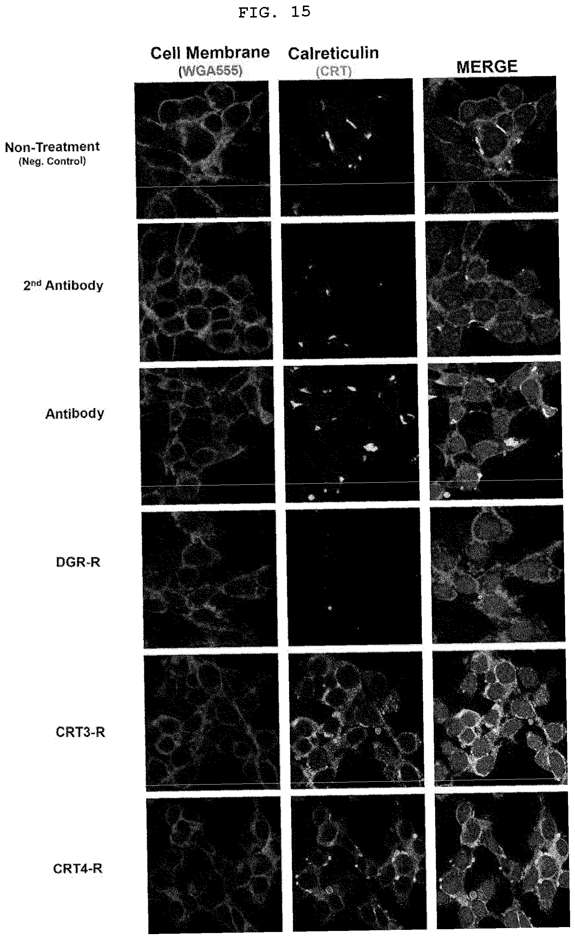

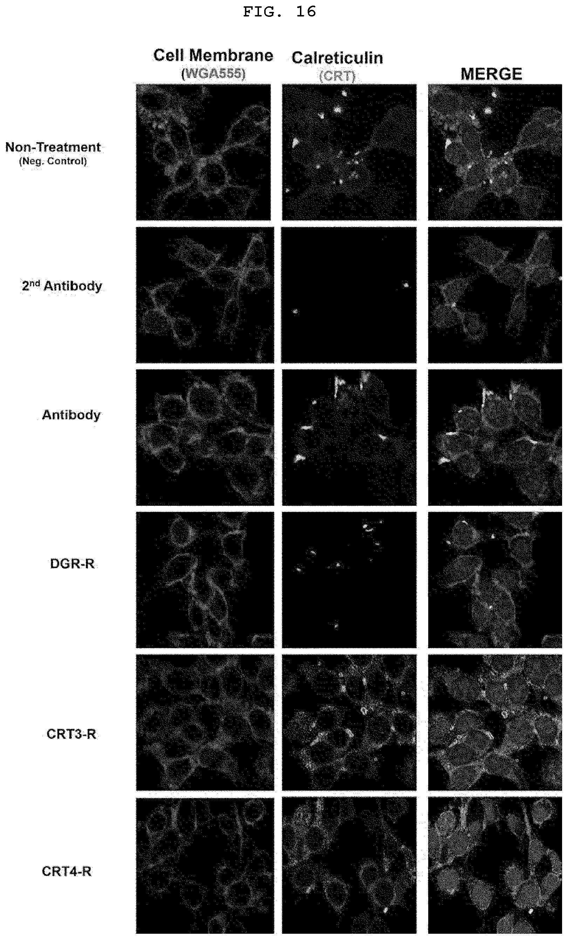

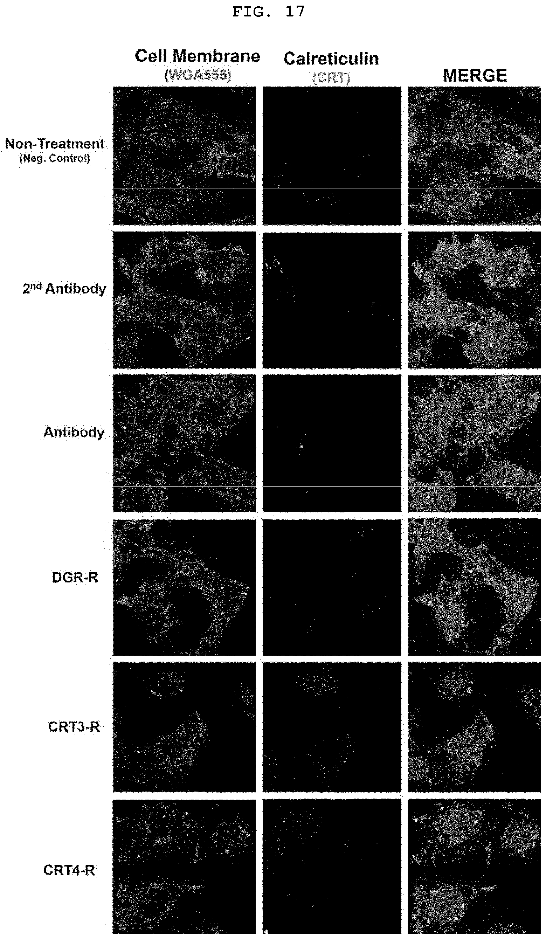

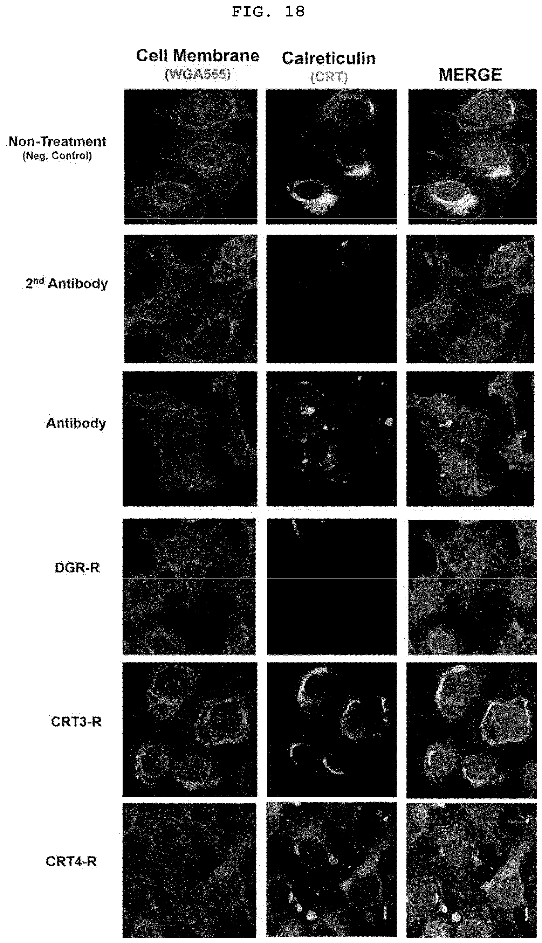

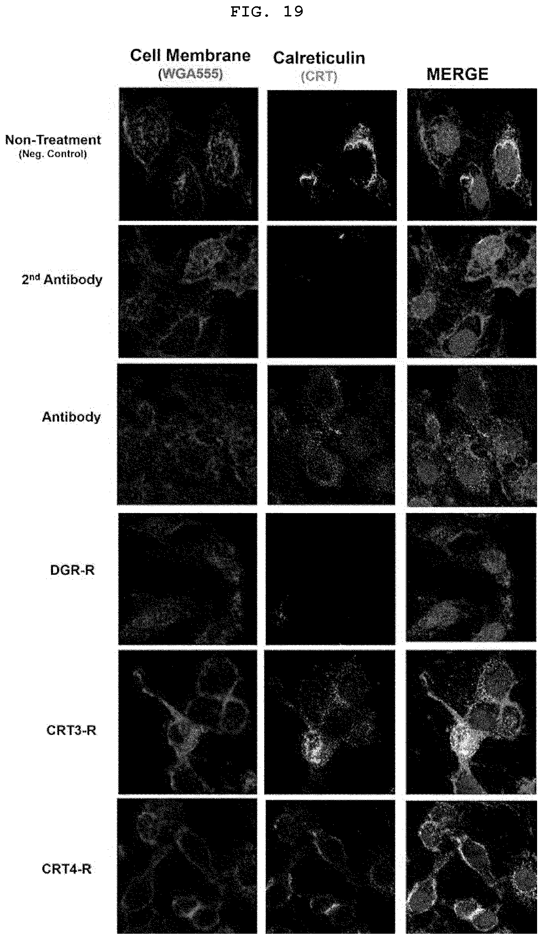

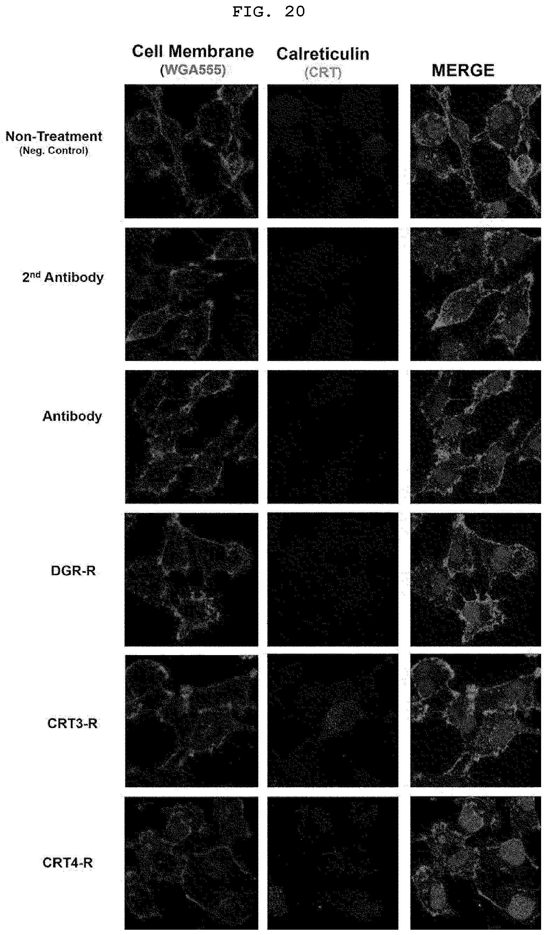

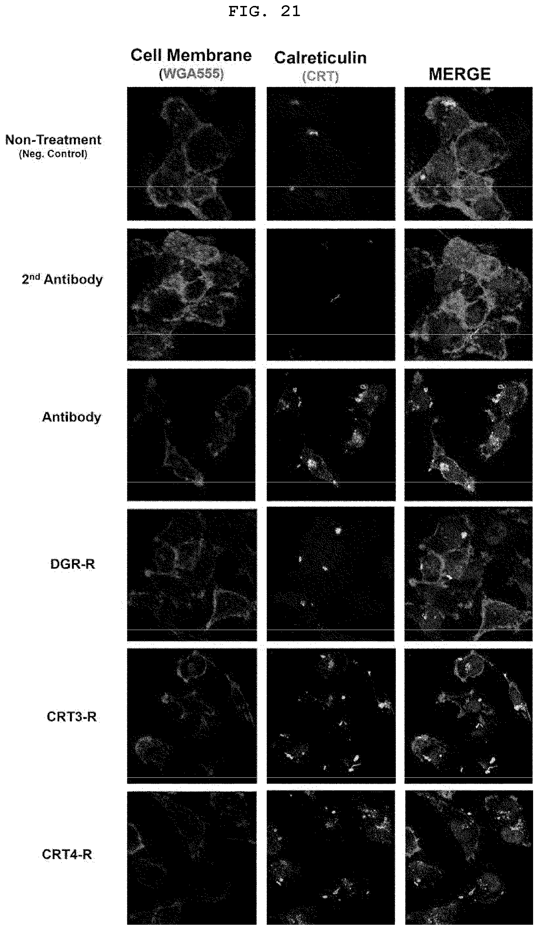

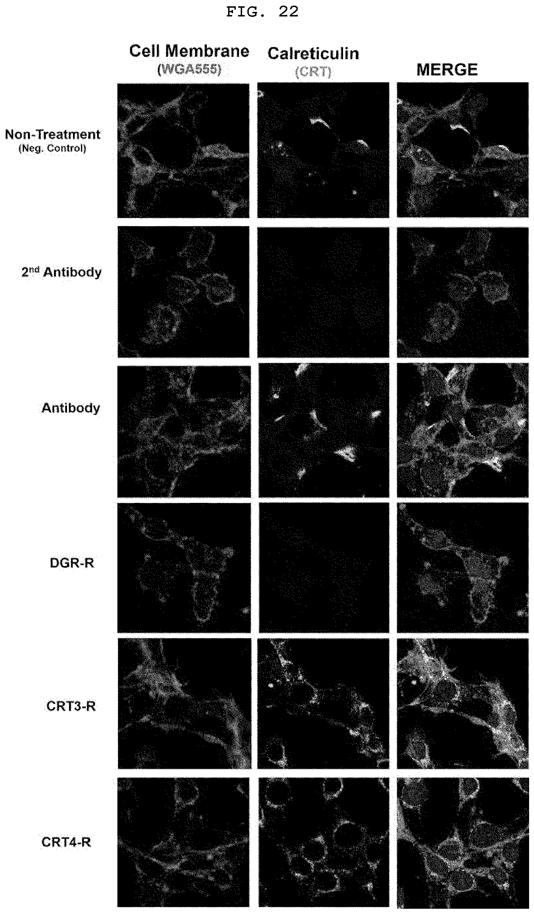

FIG. 11 is a fluorescent photograph obtained by treating CT26 (colon cancer) cells with gemcitabine and then observing the calreticulin binding force of CRT-monobody-Rluc8. Non-Treatment refers to a negative control group which is not treated with any antibody, 2^{nd} Antibody refers to an experimental group treated with only the Alexa Fluor 488 FITC secondary antibody, Antibody refers to an experimental group treated with the Alexa Fluor 488 FITC secondary antibody after CRT antibody treatment (identification of CRT exposed to cells), and DGR-R, CRT3-R, and CRT4-R refer to experimental groups treated with the His tag 488 Alexa Fluor secondary antibody after FITC antibody treatment (identification of CRT-monobody-Rluc8 bound to cells) (the experimental conditions are commonly applied to FIGS. 11 to 22 below).
FIG. 12 is a fluorescent photograph obtained by treating CT26 (colon cancer) cells with doxorubicin and then observing the calreticulin binding force of CRT-monobody-Rluc8.
FIG. 13 is a fluorescent photograph obtained by treating CT26 (colon cancer) cells with mitoxantrone and then observing the calreticulin binding force of CRT-monobody-Rluc8.
FIG. 14 is a fluorescent photograph obtained by treating MC38 (colorectal cancer) cells with gemcitabine and then observing the calreticulin binding force of CRT-monobody-Rluc8.
FIG. 15 is a fluorescent photograph obtained by treating MC38 (colorectal cancer) cells with doxorubicin and then observing the calreticulin binding force of CRT-monobody-Rluc8.
FIG. 16 is a fluorescent photograph obtained by treating MC38 (colorectal cancer) cells with mitoxantrone and then observing the calreticulin binding force of CRT-monobody-Rluc8.
FIG. 17 is a fluorescent photograph obtained by treating Hela (uterine cancer) cells with gemcitabine and then observing the calreticulin binding force of CRT-monobody-Rluc8.
FIG. 18 is a fluorescent photograph obtained by treating Hela (uterine cancer) cells with doxorubicin and then observing the calreticulin binding force of CRT-monobody-Rluc8.
FIG. 19 is a fluorescent photograph obtained by treating Hela (uterine cancer) cells with mitoxantrone and then observing the calreticulin binding force of CRT-monobody-Rluc8.
FIG. 20 is a fluorescent photograph obtained by treating MDA-MB-231 (breast cancer) cells with gemcitabine and then observing the calreticulin binding force of CRT-monobody-Rluc8.
FIG. 21 is a fluorescent photograph obtained by treating MDA-MB-231 (breast cancer) cells with doxorubicin and then observing the calreticulin binding force of CRT-monobody-Rluc8.
FIG. 22 is a fluorescent photograph obtained by treating MDA-MB-231 (breast cancer) cells with mitoxantrone and then observing the calreticulin binding force of CRT-monobody-Rluc8.
FIG. 23 is a FACS graph obtained by measuring the degree of binding of cancer cells to CRT-monobody-Rluc8 after treating CT26 cells with CRT-monobody-Rluc8. CRT Antibody is an experimental group in which cells are treated with CRT antibody and then the Alexa Fluor 488 FITC secondary antibody (identification of CRT on the cell surface), and DGR-R, CRT3-R, and CRT4-R are the experimental groups in which cells are treated with purified proteins, then the His tag antibody, and then the Alexa Fluor 488 FITC secondary antibody (identification of proteins binding to the cells).
FIG. 24 is a FACS graph obtained by measuring the degree of binding of cancer cells to CRT-monobody-Rluc8 after treating MC38 cells with CRT-monobody-Rluc8.
FIG. 25 is a FACS graph obtained by measuring the degree of binding of cancer cells to CRT-monobody-Rluc8 after treating Hela cells with CRT-monobody-Rluc8.
FIG. 26 is a FACS graph obtained by measuring the degree of binding of cancer cells to CRT-monobody-Rluc8 after treating MDA-MB-231 cells with CRT-monobody-Rluc8.
FIG. 27 is a graph obtained by analyzing in vivo stability results by measuring luciferase activity of DGR-R.
FIG. 28 is a graph obtained by analyzing in vivo stability results by measuring luciferase activity of CRT3-R.
FIG. 29 is a graph obtained by analyzing in vivo stability by measuring luciferase activity of CRT4-R.

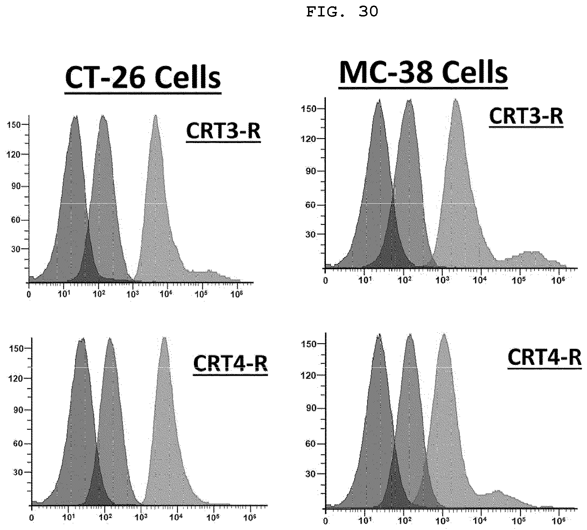

FIG. 30 is a graph obtained by analyzing whether the calreticulin-specific binding is by treating CT26 and MC38 cells with CRT-monobody-Rluc8. Under the conditions of each color, red identified CRT-monobody-Rluc8 which was bound to the cells by treating the cells with CRT-monobody-Rluc8 and then with the His tag antibody and Alexa Fluor 488 FITC secondary antibody. Blue identified CRT-monobody-Rluc8 which was bound to the cells by treating the cells with recombinant CRT protein and CRT-monobody-Rluc8, and then with the His tag antibody and Alexa Fluor 488 FITC secondary antibody. Gray identified Scrambled monobody which was bound to the cells by treating the cells with DGR-R protein (Scrambled), and then with the His tag antibody and AlexaFluor 488 FITC secondary antibody (the experimental conditions are commonly applied to FIGS. 30 to 31).
FIG. 31 is a graph obtained by analyzing whether the calreticulin-specific binding is by treating Hela and MDA-MB-231 cells with CRT-monobody-Rluc8.
FIG. 32 is a luminescence photograph for observing whether or not to bind to calreticulin by injecting CT26 cancer cells into a tumor mouse model and then injecting CRT-monobody-Rluc8 of the present invention.

### BEST MODE FOR CARRYING OUT THE INVENTION

### Definition of Terms

As used herein, the term "fibronectin" refers to a glycoprotein present in plasma and extracellular matrix (ECM), and has a structure in which multiple type I, II, and III domains having two anti-parallel beta-sheets are connected together. The fibronectin is characterized in that the type I and II domains have a disulfide bridge in the structure, but the type III domain (FnIII) has no disulfide bridge.

As used herein, the term "human fibronectin domain III (Fn3)-derived peptide" is defined as an artificial protein having a protein scaffold derived from the amino acid sequence corresponding to the 10th in the FnIII domain, and hereinafter, is abbreviated as "monobody" for convenience.

As used herein, the term "calreticulin (CRT)" refers to a major calcium storage protein in the sarcoplasmic reticulum of skeletal muscle, which binds calcium ions with low affinity but high capacity, binds about 25 calcium ions per molecule, and has a KDEL motif of the endoplasmic reticulum retention signal. Also, the calreticulin is present in the nucleus of cells and is associated with DNA binding by nuclear hormone receptors and nuclear hormone receptor mediated gene delivery. The CRT binds misfolded proteins and prevents them from being exported from the endoplasmic reticulum to the Golgi body. Recently, the importance of CRT in cancer has emerged, and when cells are damaged by anticancer drugs or the like, the damaged cells themselves expose the CRT to the cell surface to send "eat-me signals" and immunocytes remove the damaged cells. Cancer cells have characteristics of continual division and proliferation without ceasing, and as cancer progresses, especially when treated with an anticancer drug such as doxorubicin, CRT, which is a pro-phagocytic protein, increases on the cell surface, and serves to send a signal of "remove me (phagocytosis, decomposition)" to immunocytes in the blood, thereby sending a pro-phagocytic signal to macrophages.

As used herein, the term "Rluc8" refers to a renilla luciferase, which is a bioluminescent enzyme widely used as a reporter protein in molecular biosensors.

As used herein, the term "peptide conjugate" refers to a complex in which protein and drug are combined as a formulation that allows for maintaining the long-term efficacy of peptide drugs.

### DETAILED DESCRIPTION OF THE INVENTION

According to one aspect of the present invention, there is provided a recombinant monobody which specifically binds calreticulin and in which a calreticulin-binding peptide is inserted into at least one among the BC loop and the FG loop of human fibronectin domain III (Fn3).

In the recombinant monobody, the calreticulin-binding peptide may be inserted into both the BC loop and the FG loop and the calreticulin-binding peptide may consist of an amino acid sequence of SEQ ID NO: 15 or 16.

The recombinant monobody may be composed of an amino acid sequence selected from the group consisting of SEQ ID NOs: 7 to 12.

According to another aspect of the present invention, there is provided a peptide conjugate in which an anticancer compound or an anticancer protein is bound to the recombinant monobody by a covalent or non-covalent bond.

In the peptide conjugate, the anticancer compound may be at least one selected from the group consisting of doxorubicin, cyclophosphamide, mechlorethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, izabepilone, vinblastine, vincristine, vindesine, vinorelbine, estramustine, maytansine, DM1 (mertansine), DM4, dolastatin, auristatin E, auristatin F, monomethyl auristatin E, monomethyl auristatin F, and a derivative thereof, and the anticancer protein may be asparaginase, a protein toxin, an antibody specific to a cancer antigen or a fragment of the antibody, a tumor suppressor protein, or an antiangiogenic factor.

Here, the protein toxin may be botulinum toxin, tetanus toxin, shiga toxin, diphtheria toxin (DT), ricin, pseudomonas exotoxin (PE), cytolysin A (ClyA), and r-Gelonin.

The tumor suppressor protein may be von Hippel-Lindau (VHL), adenomatous polyposis coli (APC), cluster of differentiation 95 (CD95), suppression of tumorigenicity 5 (ST5), yippee like 3 (YPEL3), p53, suppression of tumorigenicity 7 (ST7), or suppression of tumorigenicity 14 (ST14).

The anticancer protein may be a protein toxin, an antibody specific to a cancer antigen or a fragment of the antibody, a tumor suppressor protein, or an antiangiogenic factor. Here, the protein toxin may be botulinum toxin, tetanus toxin, shiga toxin, diphtheria toxin (DT), ricin, pseudomonas exotoxin (PE), cytolysin A (ClyA), and r-Gelonin. The tumor suppressor protein is a protein which suppresses tumor development, and may typically include von Hippel-Lindau (VHL), adenomatous polyposis coli (APC), cluster of differentiation 95 (CD95), suppression of tumorigenicity 5 (ST5), yippee like 3 (YPEL3), p53, suppression of tumorigenicity 7 (ST7), and suppression of tumorigenicity 14 (ST14).

The antiangiogenic factor may include an anti-VEGF antibody, angiostatin, endostatin, and a kringle V domain of apolipoprotein. In addition, the anticancer protein may be a toxin, an antibody specific to a cancer antigen or a fragment of the antibody, a tumor suppressor protein, or an antiangiogenic factor.

According to further another aspect of the present invention, there is provided a pharmaceutical composition for treating cancer containing the recombinant monobody or the peptide conjugate as an active ingredient.

In the composition, the cancer may be lung cancer, gastric cancer, liver cancer, bone cancer, pancreatic cancer, gallbladder cancer, cholangioma, skin cancer, head and neck cancer, skin melanoma, uterine cancer, ovarian cancer, rectal cancer, colorectal cancer, colon cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, or thyroid cancer.

According to still another aspect of the present invention, there is provided a peptide conjugate in which a compound for contrast is bound to the recombinant monobody by a covalent or non-covalent bond.

In the peptide conjugate, the compound for contrast may be a fluorescent protein, a luminescent protein, a fluorescent molecule, a radionuclide-containing contrast agent for positron emission tomography (PET) imaging, a radionuclide-containing contrast agent for single photon emission computer tomography (SPECT) imaging, or a magnetic resonance imaging (MRI) contrast agent. Here, the fluorescent protein may be a green fluorescent protein (GFP), a yellow fluorescent protein (YFP), a red fluorescent protein (RFP), an orange fluorescent protein (OFP), a cyan fluorescent protein (CFP), a blue fluorescent protein (BFP), a far-red fluorescent protein, or a tetracysteine motif. Here, the green fluorescent protein may be an enhanced green fluorescent protein (EGFP), Emerald (Tsien, Annu. Rev. Biochem., 67: 509-544, 1998), Superfolder (Pedelacq et al., Nat. Biotech., 24: 79-88, 2006), GFP (Prendergast et al., Biochem., 17(17): 3448-3453, 1978), Azami Green (Karasawa, et al., J. Biol. Chem., 278: 34167-34171, 2003), TagGFP (Evrogen, Russia), TurboGFP (Shagin et al., Mol. Biol. Evol., 21(5): 841-850, 2004), ZsGreen (Matz et al., Nat. Biotechnol., 17: 969-973, 1999), or T-Sapphire (Zapata-Hommer et al., BMC Biotechnol., 3:5, 2003), the yellow fluorescent protein may be an enhanced yellow fluorescent protein (EYFP, Tsien, Annu. Rev. Biochem., 67: 509-544, 1998), Topaz (Hat et al., Ann. N. Y. Acad. Sci., 1: 627-633, 2002), Venus (Nagai et al., Nat. Biotechnol., 20(1): 87-90, 2002), mCitrine (Griesbeck et al., J. Biol. Chem., 276: 29188-29194, 2001), Ypet (Nguyet and Daugherty, Nat. Biotechnol., 23(3): 355-360, 2005), TagYFP (Evrogen, Russia), PhiYFP (Shagin et al., Mol. Biol. Evol., 21(5): 841-850, 2004), ZsYellow1 (Matz et al., Nat. Biotechnol., 17: 969-973, 1999), or mBanana (Shaner et al. , Nat. Biotechnol., 22: 1567-1572, 2004), the red fluorescent protein may be mRuby (Kredel et al., PLoS ONE, 4(2):e4391, 2009), mApple (Shaner et al., Nat. Methods, 5(6): 545-551, 2008), mStrawberry (Shaner et al., Nat. Biotechnol., 22: 1567-1572, 2004), AsRed2 (Shanner et al., Nat. Biotehcnol., 22: 1567-1572, 2004), or mRFP (Campbell et al., Proc. Natl. Acad. Sci. USA, 99(12): 7877-7882, 2002), the orange fluorescent protein may be Kusabira Orange (Karawawa et al., Biochem. J., 381(Pt 1): 307-312, 2004), Kusabira Orange2 (MBL International Corp., Japan), mOrange (Shaner et al., Nat. Biotechnol., 22: 1567-1572, 2004), mOrange2 (Shaner et al., Nat. Biotechnol., 22: 1567-1572, 2004), dTomato (Shaner et al., Nat. Biotechnol., 22: 1567-1572, 2004), dTomato-Tandem (Shaner et al., Nat. Biotechnol., 22: 1567-1572, 2004), TagRFP (Merzlyak et al., Nat. Methods, 4(7): 555-557, 2007), TagRFP-T (Shaner et al., Nat. Methods, 5(6): 545-551, 2008), DsRed (Baird et al., Proc. Natl. Acad. Sci. USA, 97: 11984-11989, 1999), DsRed2 (Clontech, USA), DsRed-Express (Clontech, USA), DsRed-Monomer (Clontech, USA), or mTangerine (Shaner et al., Nat. Biotechnol., 22: 1567-1572, 2004), the cyan fluorescent protein may be an enhanced cyan fluorescent protein (ECFP, Cubitt et al., Trends Biochem. Sci., 20: 448-455, 1995), mECFP (Ai et al., Biochem. J., 400(3): 531-540, 2006), mCerulean (Koushik et al., Biophys. J., 91(12): L99-L101, 2006), CyPet (Nguyet and Daugherty, Nat. Biotechnol., 23(3): 355-360, 2005), AmCyan1 (Matz et al., Nat. Biotechnol., 17: 969-973, 1999), Midori-Ishi Cyan (Karawawa et al., Biochem. J., 381(Pt 1): 307-312, 2004), TagCFP (Evrogen, Russia), or mTFP1 (Ai et al., Biochem. J., 400(3): 531-540, 2006), the blue fluorescent protein may be an enhanced blue fluorescent protein (EBFP, Clontech, USA), EBFP2 (Ai et al., Biochemistry, 46(20): 5904-5910, 2007), Azurite (Mena et al., Nat. Biotechnol., 24: 1569-1571, 2006), or mTagBFP (Subach et al., Chem. Biol., 15(10: 1116-1124, 2008), the far-red fluorescent protein may be mPlum (Wang et al., Proc. Natl. Acad. Sci. USA, 101: 16745-16749, 2004), mCherry (Shanner et al., Nat. Biotehcnol., 22: 1567-1572, 2004), dKeima-Tandem (Kogure et al. Methods, 45(3): 223-226, 2008), Jred (Shagin et al., Mol. Biol. Evol., 21(5): 841-850, 2004), mRaspberry (Shanner et al., Nat. Biotehcnol., 22: 1567-1572, 2004), HcRed1 (Fradkov et al., Biochem. J., 368(Pt 1): 17-21, 2002), HcRed-Tandem (Fradkov et al., Nat. Biotechnol., 22(3): 289-296, 2004), or AQ143 (Shkrob et al., Biochem. J., 392: 649-654, 2005), and the tetracysteine motif may be a polypeptide including a sequence of Cys-Cys-Xaa-Xaa-Cys-Cys (SEQ ID NO: 21), and the Xaa may be an amino acid except cysteine.

In the composition for contrasting a tumor, the fluorescent molecule may be one or more selected from the group consisting of Xanthene derivatives selected from the group consisting of fluorescein, fluorescein isothiocyanate (FITC), Oregon green, and Texas red; cyanine derivatives selected from the group consisting of Cy2, Cy3, Cy3B, Cy3.5, Cy5, Cy5.5, Cy7, indocarbocyanine, rhodamine, oxacarbocyanine, thiacarbocyanine, and merocyanine; oxadiazole derivatives selected from the group consisting of pyrodyloxazole, nitrobenzoxadiazole, and benzoxadiazole; acridine derivatives selected from the group consisting of Nile red, Nile orange, and acridine yellow; arylmethine derivatives selected from the group consisting of aumarine, crystal violet, and malachite green; tetrapyrrole derivatives selected from the group consisting of porphin, phthalocyanine, and bilirubin; and NIR fluorophore selected from the group consisting of X-SIGHT, Pz 247, DyLight 750, DyLight 800, Alexa Fluor 680, Alexa Fluor 750, IRDye 680, IRDye 800CW, indocyanine green, and zwitterionic near-infrared fluorophores. In particular, the NIR fluorophore is a molecule suitable for *in vivo* fluorescence imaging of large-sized animals such as humans because the NIR fluorophore can image a biomolecule lying deeper than the typical fluorescent molecules emitting light in the visible region.

In the composition for contrasting a tumor, as the radionuclide-containing contrast agent for PET imaging, a compound containing any radionuclide used for PET imaging may be used, and for example, a radionuclide having a short half-life such as C-11, N-13, 0-15, F-18, Ru-82, Ga-68, Cu-60, Cu-61, Cu-62, or Cu-64 may be used as the radionuclide, C-11, N-13, 0-15, and F-18 may be included as a constituent element in any organic compound that is easily bound to a peptide to be labeled with the peptide, and the most frequently used F-18 may be labeled on the peptide or protein by various methods known in the art, and a representative example is a one-step F-18 labeling method in which 4-nitro-3-trifluoromethyl arene is used as a precursor for F-18 labeling (Jacobson et al., Bioconjug. Chem., 22(3): 422-428, 2011). The metal radionuclides such as Ru-82, Ga-68, and Cu-64 may be covalently bonded to proteins in the form of complexes with chelators such as diethylenetriamimepentaacetic acid (DTPA), ethylenediaminetetraacetic acid (EDTA), 1,4,7,10-tetraazacyclodocecane-N,N"",N"',N""-tetraacetic acid (DOTA), 1,4,7,-triazacyclododecane-N,N",N"',N""-tetraacetic acid (NOTA), 2-[Bis[2- [bis(carboxymethyl)amino]ethyl]amino] acetic acid (DPTA), bis(thiosemi-carbazone) (BTS), propyleneamine oxime (PnAO), diaminedthiol (DADT), mercapto-acetylglycylglycylglycine (MAG3), and 6-amino-6-methylperhydro-1,4-diazepine-tetraacetic acid (AAZTA), and thus may be labeled on the recombinant monobody according to an embodiment of the present invention.

According to yet another aspect of the present invention, there is provided a composition for diagnosing or contrasting cancer containing the peptide conjugate as an active ingredient.

In the composition, the cancer may be lung cancer, gastric cancer, liver cancer, bone cancer, pancreatic cancer, gallbladder cancer, cholangioma, skin cancer, head and neck cancer, skin melanoma, uterine cancer, ovarian cancer, rectal cancer, colorectal cancer, colon cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, or thyroid cancer.

According to still yet another aspect of the present invention, there is provided a method for diagnosing a tumor in an animal except a human, the method including: administering the composition for diagnosing or contrasting cancer to an individual; and obtaining an image by means of the composition. In this case, the individual may be a human or a mammal except a human.

According to even another aspect of the present invention, there is provided a method for contrasting cancer tissues during the surgery of a mammal except a human, the method including: administering the composition for diagnosing or contrasting cancer to an individual; obtaining an image by means of the composition; and resecting an area in which a signal is shown by the composition.

According to still another embodiment of the present invention, there is provided a method for treating cancer, the method including administering a therapeutically effective amount of the pharmaceutical composition for treating cancer to an individual.

According to yet another aspect of the present invention, there is provided a use of a recombinant monobody which specifically binds calreticulin and in which a calreticulin-binding peptide is inserted into at least one among the BC loop and the FG loop of human fibronectin domain III (Fn3), or a peptide conjugate in which an anticancer compound or an anticancer protein is bound to the recombinant monobody by a covalent or non-covalent bond, for preparing an anticancer composition.

According to yet another aspect of the present invention, there is provided a use of a recombinant monobody which specifically binds calreticulin and in which a calreticulin-binding peptide is inserted into at least one among the BC loop and the FG loop of human fibronectin domain III (Fn3), or a peptide conjugate in which an anticancer compound or an anticancer protein is bound to the recombinant monobody by a covalent or non-covalent bond, for preparing a composition for diagnosing or contrasting cancer.

The pharmaceutical composition according to an embodiment of the present invention may be administered to the individual by parenteral administration, and the parenteral administration may be intravenous injection, intraperitoneal injection, intramuscular injection, subcutaneous injection, intraventricular injection, intracranial injection, intracerebrospinal injection, or intratumoral injection.

The pharmaceutical composition according to an embodiment of the present invention further includes an inert component including a pharmaceutically acceptable carrier. As used herein, the term "pharmaceutically acceptable carrier" refers to an ingredient except an active material of a composition, specifically, a pharmaceutical composition. Examples of the pharmaceutically acceptable carrier include binders, disintegrants, diluents, fillers, glidants, solubilizers or emulsifiers and salts.

In addition, the pharmaceutical composition according to an embodiment of the present invention may be administered at a dose of 0.1 mg/kg to 1 g/kg, and more preferably, administered at a dose of 0.1 mg/kg to 500 mg/kg. Meanwhile, the dose may be appropriately adjusted according to the patient's age, sex, and condition.

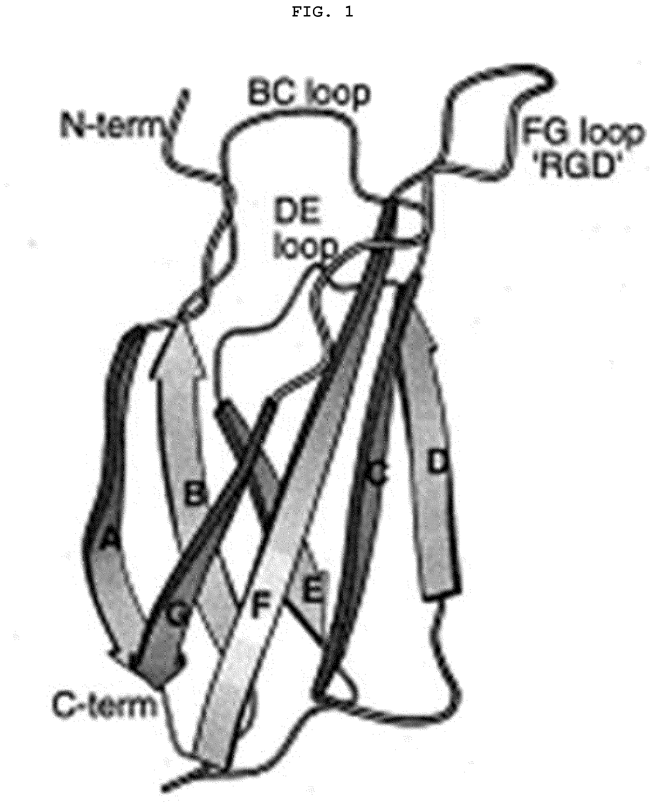

The present inventors prepared recombinant CRT-monobody by substituting two CRT peptides of Int-α (SEQ ID NO: 15) and Hep-I (SEQ ID NO: 16) at each loop position (BC, DE, and FG) of the Fn3 domain, and prepared by adding an Rluc8 gene to the C-terminus of CRT-3, CRT-4, and FN3-DGR genes for *in vivo* imaging. As a result of analyzing the expression, activity, binding force, and the like of the recombinant CRT-monobody, the novel protein specifically binding to calreticulin and having the scaffold of human fibronectin domain III with high calreticulin-binding force and excellent in vivo stability was developed (FIG. 1).

Hereinafter, the present invention will be described in more detail with reference to Examples. The present invention may, however, be embodied in different forms and should not be construed as limited to the embodiments set forth herein. Rather, these embodiments are provided so that this disclosure will be thorough and complete, and will fully convey the scope of the present invention to those skilled in the art.

### Example 1: Preparation of construct expressing monobody

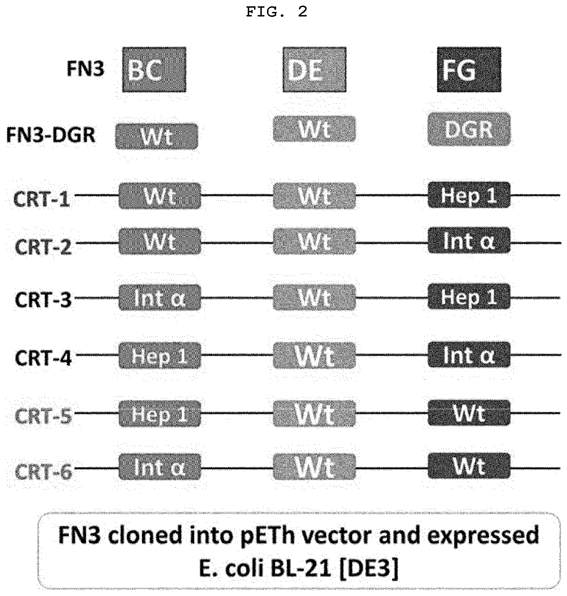

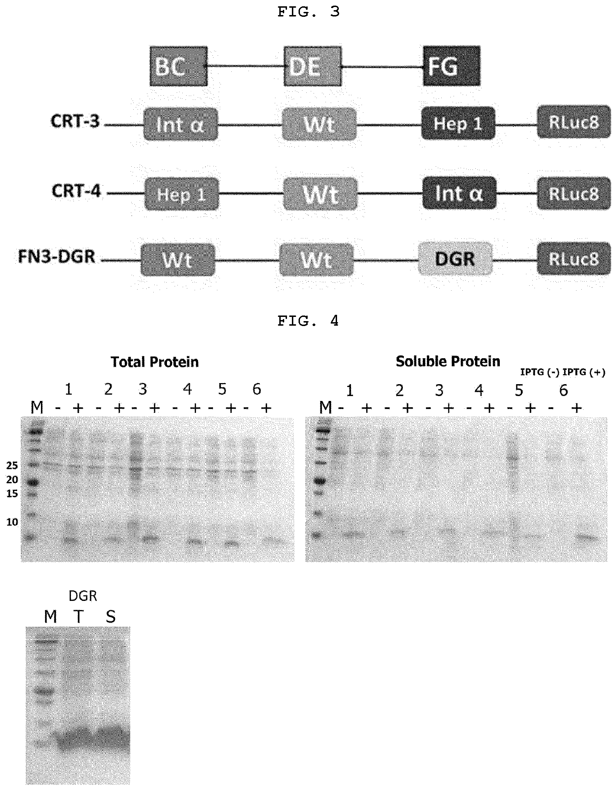

The present inventors prepared a construct expressing the monobody of the present invention. Specifically, after combining and binding peptides (Int-α and Hep-I) which specifically bind to calreticulin at each loop position of the Fn3 domain, a protein specifically binding to calreticulin was prepared. First, the gene was amplified using a forward primer (SEQ ID NO: 17) prepared to include the restriction enzyme *N*he1 site by synthesizing the inserted gene (Macrogen Inc., Korea) as shown in Table 1 below, and a reverse primer (SEQ ID NO: 18) prepared to include the restriction enzyme *Bam*H1 site (FIG. 2). Then, the amplified product was cleaved by adding restriction enzymes Nhe1 and *Bam*H1 and purified, and the gene amplification product was obtained and then introduced into pETh plasmid which was cleaved with the same restriction enzyme (PLoS One. 2017 Jul 7;12(7):e0180786), and thus pETh_CRT1, pETh_CRT2, pETh_CRT3, pETh_CRT4, pETh_CRT5, and pETh_CRT6 plasmids were prepared. In addition, a pETh_DGR plasmid was prepared as a control group by substituting an arginine-glycine-aspartate (RGD) sequence in the FG loop of the wild-type FN3 scaffold with a scramble (DGR) sequence in order to prevent integrin binding (FIG. 2). Subsequently, for *in vivo* imaging, the gene was amplified using a forward primer (SEQ ID NO: 19) and a reverse primer (SEQ ID NO: 20) of the Rluc8 gene and then purified to obtain a gene amplification product, and then the gene amplification product was introduced into pETh_CRT3, pETh_CRT4, and pETh_DGR plasmids, which have been cleaved with the BamH1 restriction enzyme, by a gibson assembly method to prepare pETh_CRT3-R, pETh_CRT4-R, and pETh_DGR-R plasmids (FIG. 3). A *E.coli* BL21 (DE3) strain was transformed with the prepared plasmid, and then each transformed strain was cultured overnight using a 50 ug/mL of an LB solid medium containing kanamycin, and then the resulting colony was cultured in an LB liquid medium containing kanamycin, thereby being used in the experiment. The information on the prepared CRT-monobody configuration is summarized in Table 1 below, and the information on the primer sequences is summarized in Table 2 below.

**[Table 1]**

| CRT-monobody configuration information | | | |
|---|---|---|---|
| Gene | Sequence substituted at Fn3 gene loop | | |
| | BC loop | DE loop | FG loop |
| CRT1 | - | - | Hep 1 |
| CRT2 | - | - | Int α |
| CRT3 | Int α | - | Hep 1 |
| CRT4 | Hep 1 | - | Int α |
| CRT5 | Hep 1 | - | - |
| CRT6 | Int α | - | - |
| DGR | - | - | DGR |

**[Table 2]**

| Primer sequence information | | |
|---|---|---|
| Primer | Base sequence (5'3') | Base sequence |
| Nhe1 | TACATATGGCTAGCGTTTCTGATGTTCCGAG | 17 |
| BamH1 | TACTGAGTGGATCCTGTTCGGTAATTAATGGAA ATTGG | 18 |
| Rluc8 F | TCCATTAATTACCGAACAGGTAGCGGCAGCGGT AGCGCTTCCAAGGTGTACGACCCC | 19 |
| Rluc8 R | TGATGATGGTGATGGTGGGATCCCTGCTCGTTC TTCAGCACGCG | 20 |

### Example 2: Protein expression level of recombinant CRT-monobody

The present inventors investigated protein expression levels of the recombinant CRT-monobodies (CRT1, CRT2, CRT3, CRT4, CRT5, and CRT6) and scrambled monobody (DGR) prepared according to Example 1. First, the recombinant *E. coli* colonies were cultured overnight in an LB liquid medium containing kanamycin, and then diluted at a ratio of 1:100 using a fresh LB medium. Thereafter, when the OD₆₀₀ value reached 0.5 to 0.7 through additional culture, isopropyl β-D-1-thiogalactopyranoside (IPTG) having a final concentration of 0.2 mM was added to the culture medium, and the culture was performed in a shaking incubator at 200 rpm and 37 °C. The cultured recombinant *E. coli* was boiled at 95 °C for 10 minutes after adding an SDS-PAGE sample buffer based on OD4, and then loaded on SDS-PAGE to confirm the protein expression level.

As a result, it was confirmed that gene overexpression was induced by IPTG, and it was found that the expressed gene had solubility (FIG. 4).

### Example 3: Purification of CRT-monobody

The present inventors cultured the recombinant strain by the method of Example 2, washed the strain with PBS to remove the supernatant, and then resuspended the strain in a lysis buffer (50 mM NaH₂PO₄, 300 mM NaCl, 10 mM imidazole, and pH 8.0). Then, the resuspended strain was crushed by using a sonicator under conditions of 5 seconds/10 seconds (on/off stop) and 10 minutes, the crushed strain was centrifuged (10,000 × g, 40 minutes, and 4 degrees), the supernatant was loaded on a Ni-NTA spin column (Qiagen), and the CRT-monobody was bound to Ni-NTA by centrifugation (890 × g and 1 minute). The unbound proteins were then removed by loading a wash buffer (50 mM NaH₂PO₄, 300 mM NaCl, 20 mM imidazole, and pH 8.0) into a column and 300 uL of elution buffer (50 mM NaH₂PO₄, 300 mM NaCl, 500 mM imidazole, and pH 8.0) was loaded into the column to elute his tag-proteins. From the eluted proteins, imidazole was removed using a dialysis membrane kit (66380, Thermo Fisher Scientific), followed by addition of an SDS-PAGE sample buffer, boiling the resultant mixture at 95 °C for 10 minutes, and then loading the mixture on SDS-PAGE to confirm the protein purification degree.

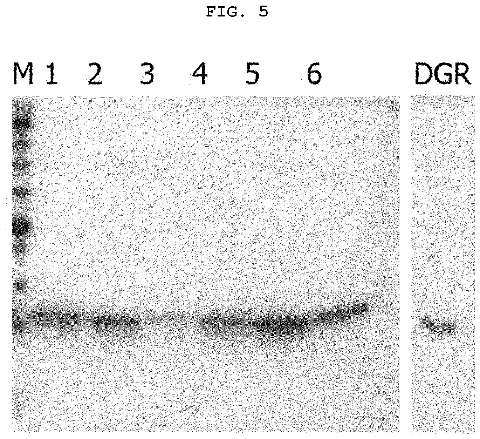

As a result, all the purified proteins were purified with a purity of at least 95%, which was used in the experiment (FIG. 5).

### Example 4: Analysis of binding force of CRT-monobody to calreticulin

In order to verify the binding force of the purified CRT-monobody to calreticulin, the present inventors observed the degree of binding with a confocal microscope after treating the cancer cells with CRT-monobody. Specifically, CT26 (colon cancer) cells, which are cancer cells, were seeded at 1×10⁵ cells per well in chamber slides (154534PAK, Thermo Fisher Scientific) and cultured overnight in a C0₂ incubator. Then, each of the cells was treated with mitoxantrone (25 µM), which is an anticancer drug, to induce calreticulin on the surface thereof, treated with gemcitabine (15 µM) as a control group, and cultured for 4 hours, washed with PBS, and reacted with 3% paraformaldehyde for 10 minutes to fix the cells. Then, a block buffer (3% BSA in PBS) was treated for 1 hour to prevent the hard-to-bind antibody, and the CRT-monobody (10 µg/mL) of the present invention was treated in each well and reacted for 1 hour to induce binding to calreticulin. Subsequently, anti-his tag primary antibodies (1:5000, 3% BSA in PBS) were treated for identifying CRT-monobody, CRT antibodies (1:500, 3% BSA in PBS) were treated for identifying calreticulin, and Alexa 488 secondary antibodies (1:500, 3% BSA in PBS) were treated for imaging. In addition, Alexa 555 conjugated WGA-555 (1:2000, 3% BSA in PBS) was treated in order to distinguish cell membranes, DAPI-Glod-antifade was treated, and mounting was performed, and the prepared slides were refrigerated under dark conditions.

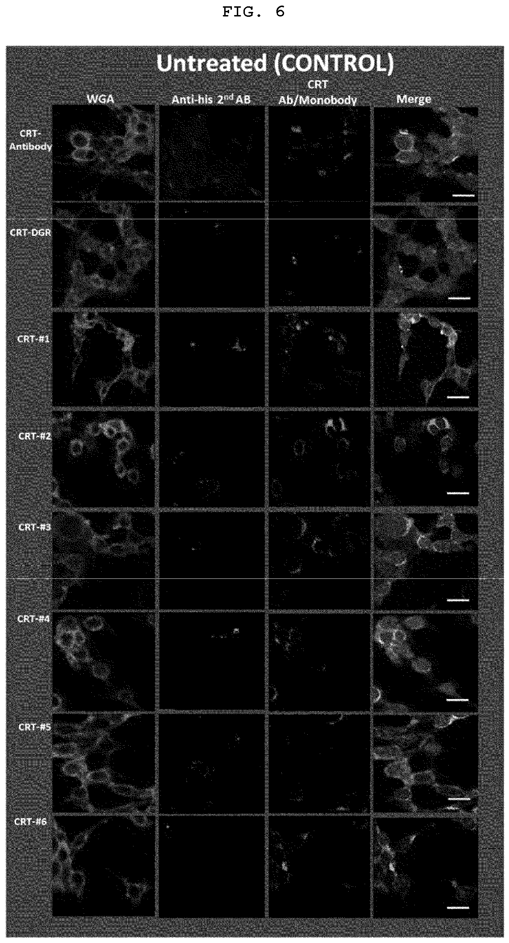

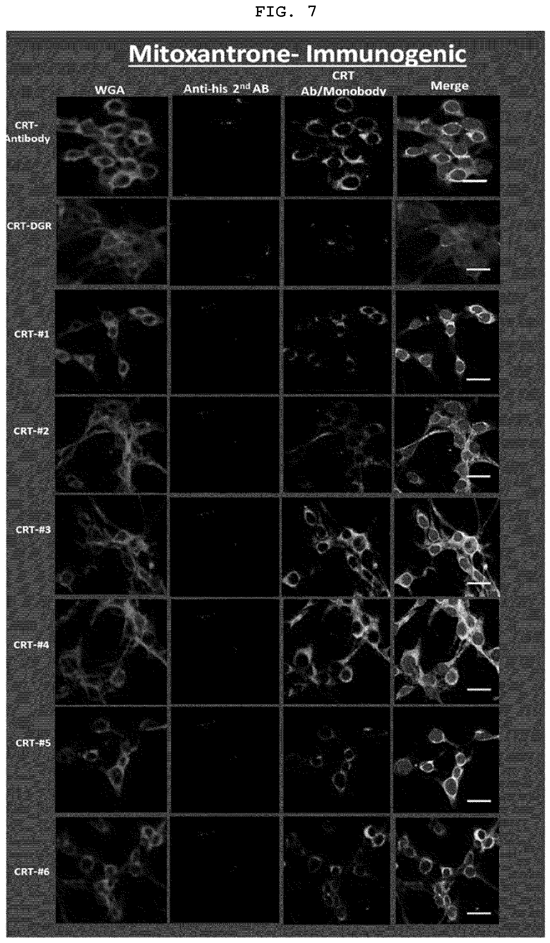

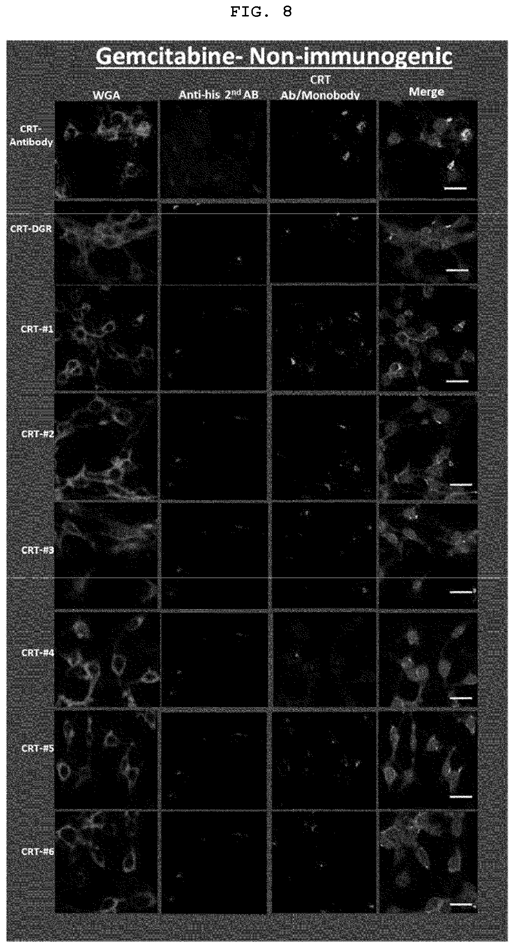

As a result, it was confirmed that the cells which were not treated with the anticancer drug and the cells which were treated with gemcitabine did not expose calreticulin on the cell membrane, and it was confirmed that CRT-monobody did not bind to the cells. However, it was confirmed that the cells which were treated with mitoxantrone exposed calreticulin on the cell membrane, and the DGR protein did not bind to the cell surface, but the CRT-monobody bound to the cell surface. In particular, it was found that CRT3 and CRT4 proteins were more strongly bound to the cells in which calreticulin was exposed on the cell membrane than other CRT-monobodies (FIGS. 6 to 8). Subsequently, CRT3 and CRT4 having the best binding force were selected, and the Rluc8 gene was fused with CRT3 and CRT4 and used in the experiments for *in vivo* experiments.

### Example 5: Protein expression level of recombinant CRT-monobody-Rluc8

The present inventors investigated protein expression levels of the recombinant CRT-monobody-Rluc8 (CRT3-R and CRT4-R) and scrambled monobody-Rluc8 (DGR-R) prepared according to Example 1. The recombinant *E. coli* culture medium cultured as in Example 2 was boiled at 95 °C for 10 minutes after adding an SDS-PAGE sample buffer based on OD4, and then loaded on SDS-PAGE to confirm the protein expression level.

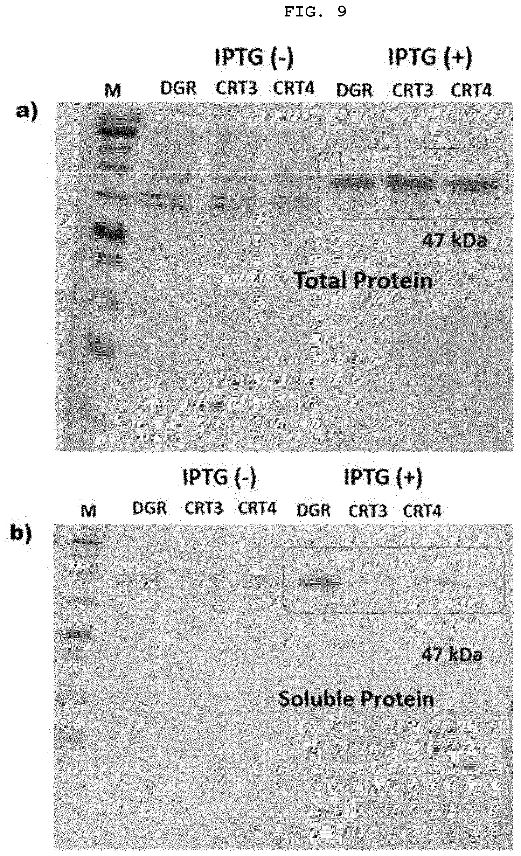

As a result, it was confirmed that gene overexpression was induced by IPTG, and it was found that the expressed gene had solubility. In addition, it was confirmed that the protein structure was stably maintained because there was no truncated size of the CRT-monobody-Rluc8 protein (FIG. 9).

### Example 6: Analysis of reporter protein activity

The present inventors resuspended 1 mL of the strains (CRT3-R, CRT4-R, and DGR-R) cultured in Example 2 in PBS in order to analyze the activity of reporter protein. Then, 50 uL of coelenterazine (1 µg/mL) diluted in ethanol was added, as a substrate, to the resuspended strain, and then luciferase activity values were imaged under the condition of 1 second exposure time using a NightOWL II LB 983 *in vivo* imaging system (Berthold technologies, GmbH & Co. KG, Germany).

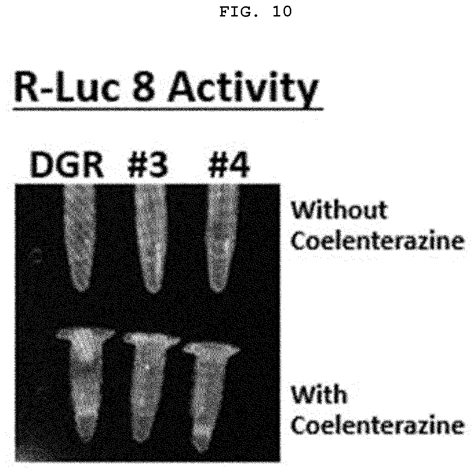

As a result, in the recombinant strains of which the luminescence was not identified before the addition of coelenterazine, the luminescence signals were identified after the addition of the substrate. This suggests that the Rluc8-fused monobody exhibits luciferase activity (FIG. 10).

### Example 7: Analysis of binding force of recombinant CRT-monobody-Rluc8 to calreticulin

In order to verify the binding force of the CRT-monobody-Rluc8 protein to calreticulin, the present inventors observed the degree of binding with a confocal microscope after purifying CRT3-R, CRT4-R, and DGR-R as in Example 3 and treating cancer cells with the CRT-monobody-Rluc8 proteins. Specifically, cancer cells such as CT26 (colon cancer), MC38 (colon cancer), Hela (uterine cancer), and MDA-MB-231 (breast cancer) were seeded at 1×10⁵ cells per well in chamber slides (154534PAK, Thermo Fisher Scientific) and cultured overnight in a C0₂ incubator. Then, each of the cells was treated with doxorubicin (25 µM) and mitoxantrone (25 µM), which are anticancer drugs, to induce calreticulin on the surface thereof, treated with gemcitabine (15 µM) as a control group, and cultured for 4 hours, washed with PBS, and reacted with 3% paraformaldehyde for 10 minutes to fix the cells. Then, a block buffer (3% BSA in PBS) was treated for 1 hour to prevent the hard-to-bind antibody, and the CRT-monobody (10 µg/mL) was treated in each well and reacted for 1 hour to induce binding to calreticulin. Subsequently, anti-his tag primary antibodies (1:5000, 3% BSA in PBS) were treated for identifying CRT-monobody, CRT antibodies (1:500, 3% BSA in PBS) were treated for identifying calreticulin, and Alexa 488 secondary antibodies (1:500, 3% BSA in PBS) were treated for imaging. In addition, Alexa 555 conjugated WGA-555 (1:2000, 3% BSA in PBS) was treated in order to distinguish cell membranes, DAPI-Glod-antifade was treated, and mounting was performed, and the prepared slides were refrigerated under dark conditions.

As a result, it was confirmed that when the CT26 (colon cancer), MC38 (colon cancer), Hela (uterine cancer), and MDA-MB-231 (breast cancer) cells were not treated with an anticancer drug and when the cells were treated with gemcitabine, there was no exposure of calreticulin on the cell membrane, and it was confirmed that CRT-monobody did not bind to the cells. However, it was confirmed that the cells treated with mitoxantrone and doxorubicin exposed calreticulin on the cell membrane, and the DGR-R protein did not bind to the cell surface, but the CRT3-R and CRT4-R proteins bind to the cell surface (FIGS. 11 to 22).

### Example 8: Flow cytometry

The present inventors allowed cancer cells to react with CRT-monobody-Rluc8, and then measured the binding degree in the number of cells in order to more accurately confirm the binding force of CRT-monobody-Rluc8, which was purified according to an embodiment of the present invention, to calreticulin. To this end, CT26, MC38, Hela, and MDA-MB-231 cells were first cultured, and then the expression of calreticulin on the cell surface was induced by the same method (gemcitabine, doxorubicin, and mitoxantrone) as in Example 5. First, the cells were washed with PBS, and then the attached cells were obtained, and reacted with 3% paraformaldehyde for 10 minutes to fix the cells, and a block buffer (3% BSA in PBS) was treated for 1 hour to prevent the hard-to-bind antibody. Then, CRT-monobody (5 µg/mL) was treated in each well and reacted for 1 hour to induce binding to calreticulin. Subsequently, anti-his tag primary antibodies (1:5000, 3% BSA in PBS) were treated for identifying CRT-monobody, CRT antibodies (1:500, 3% BSA in PBS) were treated for identifying calreticulin, and Alexa 488 secondary antibodies (1:500, 3% BSA in PBS) were treated. Finally, in order to identify cells bound to CRT-monobody, fluorescence signals of the cells were identified using a flow cytometer (BD Bioscience).

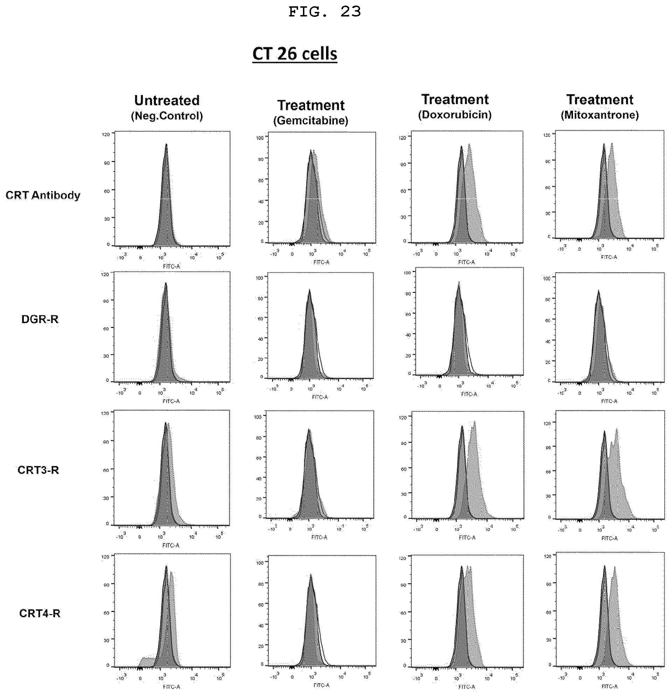

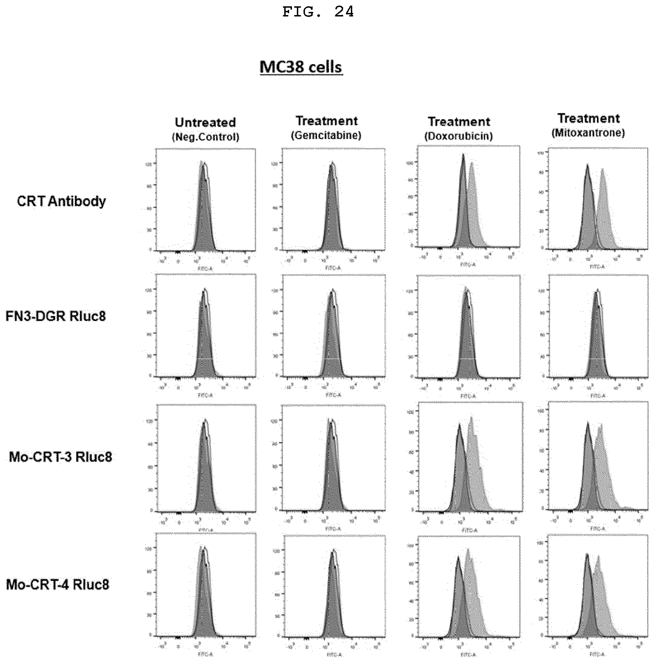

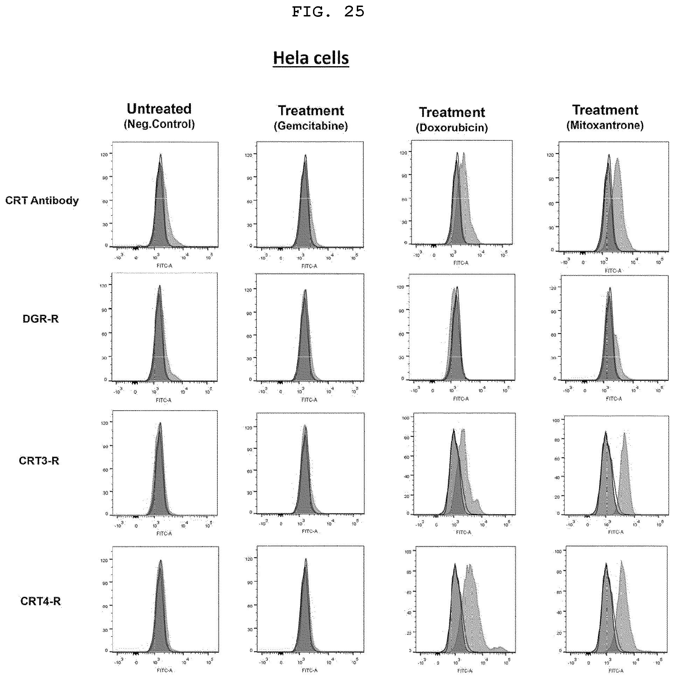

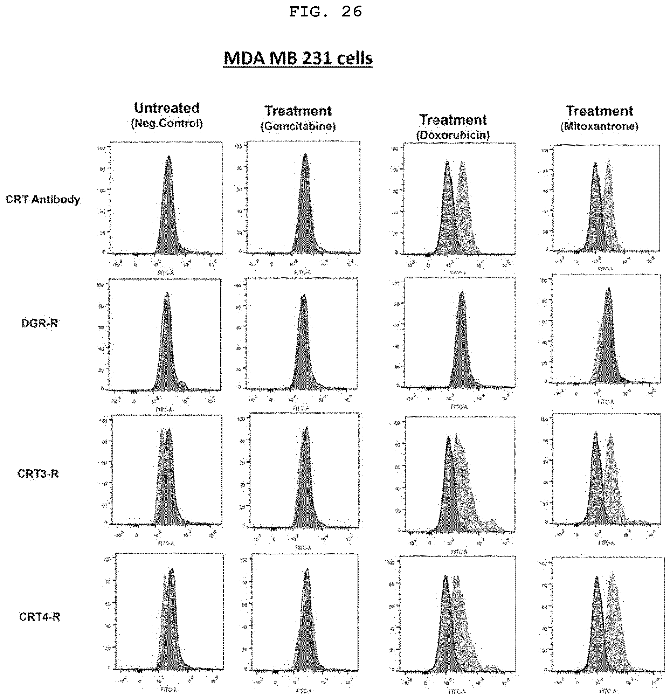

As a result, when the CT26 (colon cancer), MC38 (colon cancer), Hela (uterine cancer), and MDA-MB-231 (breast cancer) cells were not treated with an anticancer drug and when the cells were treated with gemcitabine, no exposure of calreticulin on the cell membrane was confirmed through fluorescence signals for each cell, and there was no signal change after CRT-monobody treatment of the present invention, so that it was confirmed that the CRT-monobody was not bound to the cells. However, it was found that the cells treated with mitoxantrone and doxorubicin exposed calreticulin on the cell membrane, and the DGR-R protein did not bind to the cell surface because there was no change in the signal, but the CRT3-R and CRT4-R proteins were bound to the cell surface because the Alexa 488 fluorescence signal was detected (FIGS. 23 to 26) .

### Example 9: Confirmation of in vivo stability

The present inventors confirmed Rluc8 activity after treating serums of mice with the CRT-monobody-Rluc8 protein of the present invention at varying points of time in order to confirm how stable the protein is *in vivo.* Specifically, after the blood of mice was collected, the blood was coagulated under a refrigeration condition and then centrifuged to separate serum. Then, the mouse serum (150 uL) was treated with purified CRT-monobody-Rluc8 (60 µg) (CRT3-R and CRT4-R) and DGR-R at varying points of time (0, 4, 12, and 24 hours), and 10 uL of coelenterazine (40 µg/mL) was treated to measure the Rluc8 activity. The serum boiled under the above conditions was used as a positive control group, and the serum was used as a negative control group.

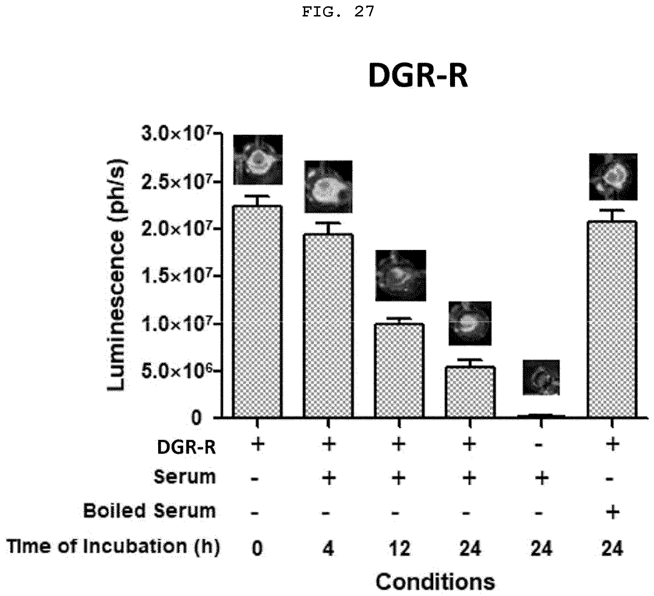

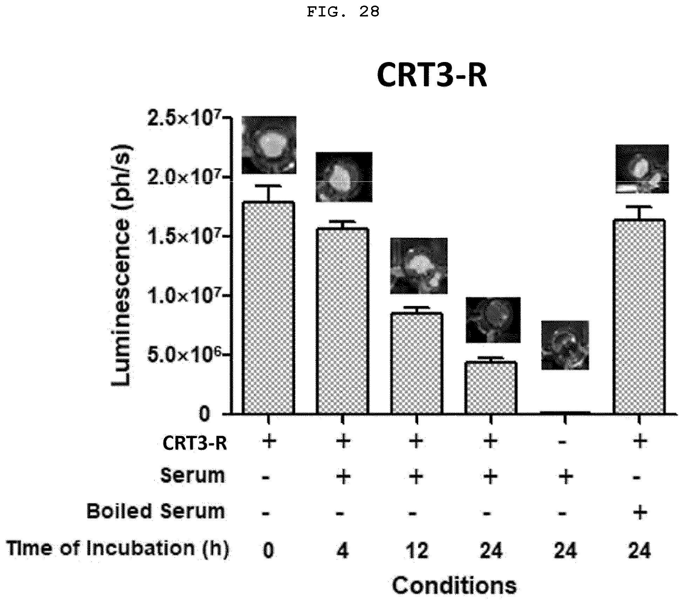

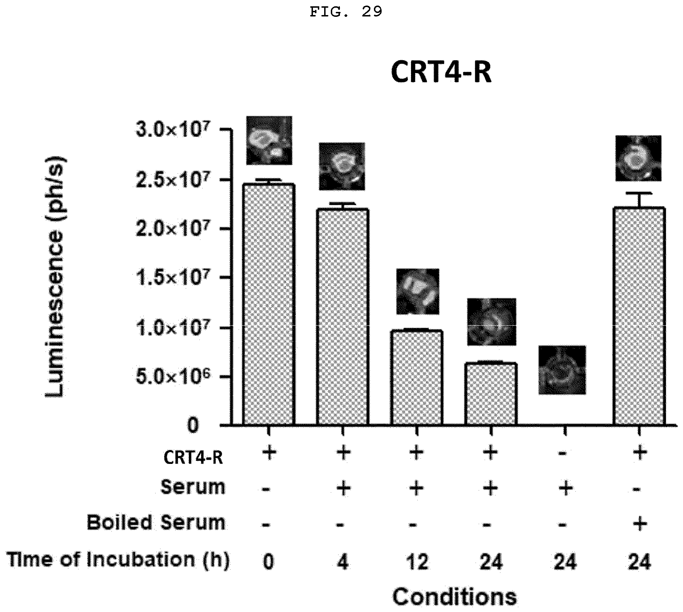

As a result, it was found that the CRT3-R, CRT4-R, and DGR-R treated in the serum maintained activity of at least 85% even after 4 hours and maintained Rluc8 activity of at least 50% even after 12 hours. The above results suggest that the CRT-monobody-Rluc8 protein of the present invention is very stable *in vivo* (FIGS. 27 to 29) .

### Example 10: Confirmation of specificity to calreticulin

The present inventors performed a competitive binding analysis in order to confirm whether the CRT-monobody-Rluc8 of the present invention specifically binds only to calreticulin (CRT) on the cell surface. First, CT26, MC38, Hela, and MDA-MB-231 cells were cultured, and then mitoxantrone (20 µM) was treated to induce the expression of calreticulin on the cell surface. Then, the drug-treated cancer cells were washed with PBS, and the attached cells were obtained, and then was reacted with 3% paraformaldehyde for 10 minutes to fix the cells, and a block buffer (3% BSA in PBS) was treated for 1 hour to prevent the hard-to-bind antibody.

Then, CRT-monobody-Rluc8 (100 µg/well) was treated and reacted for 1 hour to induce binding to calreticulin. In addition, drug-treated cancer cells were previously treated with 10 pg/well of calreticulin protein (ab15729, abcam) and then treated with CRT-monobody-Rluc8 (100 µg/well) and reacted for 1 hour to induce binding to calreticulin. Anti-his tag primary antibodies (1:5000, 3% BSA in PBS) were treated for identifying CRT-monobody-Rluc8 and Alexa 488 secondary antibodies (1:500, 3% BSA in PBS) were treated. Finally, in order to identify cells bound to CRT-monobody, fluorescence signals of the cells were identified using a flow cytometer (BD Bioscience).

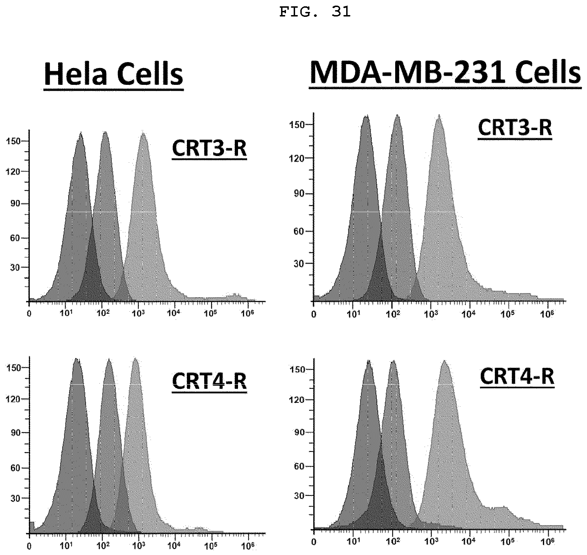

As a result, it was confirmed that the CRT3-R and CRT4-R proteins were strongly bound to CT26 (colon cancer), MC38 (colon cancer), Hela (uterine cancer), and MDA-MB-231 (breast cancer) cells in which the exposure of calreticulin was induced on the cell membrane, and thus the fluorescence signal was strongly detected, but the fluorescence signal was weakened in the group treated with the calreticulin protein. The above results suggest that the CRT3-R and CRT4-R proteins specifically bind to calreticulin as the concentration of the CRT3-R and CRT4-R proteins binding to the calreticulin on the cell membrane decreases due to the binding of the CRT3-R and CRT4-R proteins to the recombinant calreticulin protein (FIGS. 30 and 31).

### Example 11: Confirmation of calreticulin specificity in tumor animal models

The present inventors have confirmed the calreticulin specificity of CRT-monobody-Rluc8 of the present invention in tumor mouse models. Specifically, in order to confirm CRT-monobody-Rluc8 binding to calreticulin in drug-treated tumor animal models, CT26 cancer cells were subcutaneously injected into the flanks of Balb/c mice to prepare tumor animal models, and then calreticulin was induced on the surface of the tumor cells using mitoxantrone (3 mg/kg), and 6 hours after injection of CRT-monobody-Rluc8 (60 pg/100 pL), the tumor was excised, and 50 µL of coelenterazine (2 mg/mL) was treated to confirm luciferase activity in the tumor tissue.

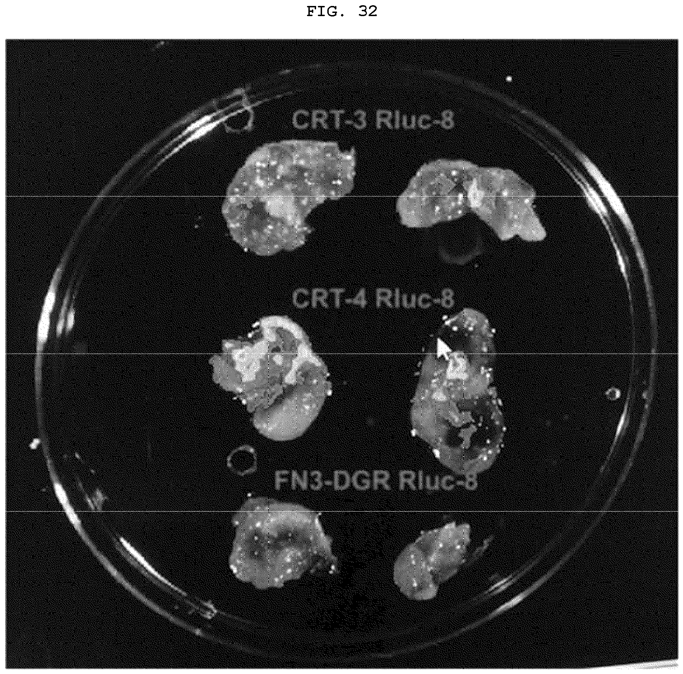

As a result, it was confirmed that the tumor tissue treated with the DGR-R protein exhibited little Rluc8 luminescence signal, whereas the tumor tissues treated with the CRT3-R and CRT4-R proteins exhibited Rluc8 luminescence signal. The above results suggest that the tumor cells that have been treated with a drug in a small-animal model may be imaged by the CRT3-R and CRT4-R proteins of the present invention (FIG. 32).

Consequently, the novel protein specifically binding to calreticulin and having the scaffold of human fibronectin domain III of the present invention has high binding force to calreticulin and excellent in vivo stability, and thus can be advantageously used as a candidate material for probes for performing an early evaluation of cancer therapy response and an evaluation of toxicity effects.

The present invention is described with reference to the described examples, but the examples are merely illustrative. Therefore, it will be understood by those skilled in the art that various modifications and other equivalent embodiments can be made from the described embodiments. Hence, the real protective scope of the present invention shall be determined by the technical scope of the accompanying claims.

## Claims

1. A recombinant monobody which specifically binds calreticulin and in which a calreticulin-binding peptide is inserted into at least one among the BC loop and the FG loop of human fibronectin domain III (Fn3).

2. The recombinant monobody of claim 1, wherein the calreticulin-binding peptide is inserted into both the BC loop and the FG loop.

3. The recombinant monobody of claim 1 or 2, wherein the calreticulin-binding peptide consists of an amino acid sequence of SEQ ID NO: 15 or 16.

4. The recombinant monobody of claim 1, wherein the recombinant monobody is composed of an amino acid sequence selected from the group consisting of SEQ ID NOs: 7 to 12.

5. A peptide conjugate in which an anticancer compound or an anticancer protein is bound to the recombinant monobody of claim 1 or 2 by a covalent or non-covalent bond.

6. The peptide conjugate of claim 5, wherein the anticancer compound is at least one selected from the group consisting of doxorubicin, cyclophosphamide, mechlorethamine, uramustine, melphalan, chlorambucil, ifosfamide, bendamustine, carmustine, lomustine, streptozocin, busulfan, dacarbazine, temozolomide, thiotepa, altretamine, duocarmycin, cisplatin, carboplatin, nedaplatin, oxaliplatin, satraplatin, triplatin tetranitrate, 5-fluorouracil, 6-mercaptopurine, capecitabine, cladribine, clofarabine, cystarbine, floxuridine, fludarabine, gemcitabine, hydroxyurea, methotrexate, pemetrexed, pentostatin, thioguanine, camptothecin, topotecan, irinotecan, etoposide, teniposide, mitoxantrone, paclitaxel, docetaxel, izabepilone, vinblastine, vincristine, vindesine, vinorelbine, estramustine, maytansine, DM1 (mertansine), DM4, dolastatin, auristatin E, auristatin F, monomethyl auristatin E, monomethyl auristatin F, and a derivative thereof.

7. The peptide conjugate of claim 5, wherein the anticancer protein is asparaginase, a protein toxin, an antibody specific to a cancer antigen or a fragment of the antibody, a tumor suppressor protein, or an antiangiogenic factor.

8. The peptide conjugate of claim 7, wherein the tumor suppressor protein is von Hippel-Lindau (VHL), adenomatous polyposis coli (APC), cluster of differentiation 95 (CD95), suppression of tumorigenicity 5 (ST5), yippee like 3 (YPEL3), p53, suppression of tumorigenicity 7 (ST7), or suppression of tumorigenicity 14 (ST14).

9. A pharmaceutical composition for treating cancer comprising, as an active ingredient, the recombinant monobody of claim 1 or 2 or the peptide conjugate of claim 5.

10. The pharmaceutical composition of claim 9, wherein the cancer is lung cancer, gastric cancer, liver cancer, bone cancer, pancreatic cancer, gallbladder cancer, cholangioma, skin cancer, head and neck cancer, skin melanoma, uterine cancer, ovarian cancer, rectal cancer, colorectal cancer, colon cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, or thyroid cancer.

11. A peptide conjugate in which a compound for contrast is bound to the recombinant monobody of claim 1 or 2 by a covalent or non-covalent bond.

12. The peptide conjugate of claim 11, wherein the compound for contrast is a fluorescent protein, a fluorescent molecule, a radionuclide-containing contrast agent for positron emission tomography (PET) imaging, a radionuclide-containing contrast agent for single photon emission computer tomography (SPECT) imaging, or a magnetic resonance imaging (MRI) contrast agent.

13. The peptide conjugate of claim 12, wherein the fluorescent protein is a green fluorescent protein (GFP), a yellow fluorescent protein (YFP), a red fluorescent protein (RFP), an orange fluorescent protein (OFP), a cyan fluorescent protein (CFP), a blue fluorescent protein (BFP), a far-red fluorescent protein, or a tetracysteine motif.

14. A composition for diagnosing or contrasting cancer comprising the peptide conjugate of claim 11 as an active ingredient.

15. The composition of claim 14, wherein the cancer is lung cancer, gastric cancer, liver cancer, bone cancer, pancreatic cancer, gallbladder cancer, cholangioma, skin cancer, head and neck cancer, skin melanoma, uterine cancer, ovarian cancer, rectal cancer, colorectal cancer, colon cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, or thyroid cancer.

16. A method for diagnosing a tumor in an animal except a human, the method comprising:
administering the composition for diagnosing or contrasting cancer to an individual; and
obtaining an image by means of the composition.

17. A method for contrasting cancer tissues during the surgery of a mammal except a human, the method comprising:
administering the composition for diagnosing or contrasting cancer to an individual;
obtaining an image by means of the composition; and
resecting an area in which a signal is shown by the composition.

18. A method for treating cancer comprising administering a therapeutically effective amount of the pharmaceutical composition for treating cancer of claim 9 to an individual.

19. A use of a recombinant monobody which specifically binds calreticulin and in which a calreticulin-binding peptide is inserted into at least one among the BC loop and the FG loop of human fibronectin domain III (Fn3), or of a peptide conjugate in which an anticancer compound or an anticancer protein is bound to the recombinant monobody by a covalent or non-covalent bond, for preparing an anticancer composition.

20. A use of a recombinant monobody which specifically binds calreticulin and in which a calreticulin-binding peptide is inserted into at least one among the BC loop and the FG loop of human fibronectin domain III (Fn3), or of a peptide conjugate in which an anticancer compound or an anticancer protein is bound to the recombinant monobody by a covalent or non-covalent bond, for preparing a composition for diagnosing or contrasting cancer.
